# EUROPEAN PATENT APPLICATION

(11) **EP 4 609 893 A1**
(43) Date of publication of application: **03.09.2025**
(21) Application number: 24160906.4
(22) Date of filing: 01.03.2024
(51) Int. Cl.: A61M 3/02, B29C 45/00, B29C 45/14, B29L 31/56

(54) **CONTAINER FOR IRRIGATION APPLICATION COMPRISING A PRECISELY DEFINED OPENING AND A PRODUCTION PROCESS THEREOF**

(71) Applicant: B. Braun Melsungen AG, 34212 Melsungen (DE)
(72) Inventor: LAMPRECHT, Volker, 34212 Melsungen (DE)
(74) Representative: Maiwald GmbH

(57) **Abstract**

A process for producing a container for irrigation application, comprising the steps of providing a base container comprising at least one wall, wherein the at least one wall comprises an opening; providing a break-off cap; fluidly connecting in a connecting step the break-off cap with the opening of said base container forming a connection of the opening with the break-off cap; sealing in a sealing step the connection of the opening with the break-off cap.

## Description

### Technical Field of the Invention

The present invention is located in the field of processing of containers for irrigation application having a precisely defined opening, the process of producing such a container, the use of such containers in irrigation application, and a method for irrigating an object during surgery with such a container.

### Background of the Invention

Irrigation containers are usually used to drain and wash surgery fields, such as body cavities, tissues, wounds, and burns, during surgeries. Typically, irrigation containers are bottles comprising a sterile liquid, wherein the bottle should be openable by hand. Usually, during operation, the sterile liquid is poured out thereby ensuring that the sterile status of the liquid is kept intact up until the liquid contacts the surgery field. Nowadays, normally blown bottles made from plastic material are used to achieve this simple functionality. Thus, usually, the filled containers are produced in a blow-fill process, wherein the process comprises the production of the actual container by blow-molding, the filling of the container and the sealing of the container. Hence, in the blow-molding process, usually a parison made from thermoplastic material is blown into a mold and the sealing step comprises sealing the opening, which is therefore also made from the thermoplastic material, into a closure. The closure is usually formed to assist in opening the container.

### Summary of the Invention

However, the problem with this process and the resulting container is that the closure can only be formed from outside, i.e., when forming the thermoplastic material by a mold. Reason is that the closure is formed by pressing the opening together, thereby closing and sealing the opening.

Forming the closure from the outside, however, does not allow for a precise forming of the closure. However, precise forming of the closure is in particular necessary for forming closures, which are opened by breaking or by twisting and therefore require a pre-determined breaking point. Furthermore, precise forming is in particular necessary for forming closures, which have tamper evidence feature.

Hence, there is the need for a container for irrigation and a production process thereof, which allows for precise forming of the closure.

It has now been found out that by sealing a pre-produced closure, i.e., a break-off cap, to the opening, a precisely formed closure can be added to the container for irrigation.

Thus, it has been surprisingly found out in the present invention that above-mentioned object can be solved by a process for producing a container for irrigation application, comprising the steps of providing a base container comprising at least one wall, wherein the at least one wall comprises an opening; providing a break-off cap; fluidly connecting in a connecting step the break-off cap with the opening of said base container forming a connection of the opening with the break-off cap; sealing in a sealing step the connection of the opening with the break-off cap.

It has been further found out that above-mentioned object of the present invention can be achieved by a container for irrigation application, the container comprising a base container comprising at least one wall, wherein the at least one wall comprises an opening, a break-off cap fluidly connected to said opening, a seal between the break-off cap and the base container.

Furthermore, it has been found out that the object of the present invention is achieved by a use of the container according to any of the present application in an irrigation application.

Finally, it has been also found out that the object of the present invention is achieved by a method for irrigating an object during surgery with a container according to the present invention.

The advantage of the present invention is that container for irrigation can be produced, which have more precisely produced closures for i.e., break-off closures, and thus allow for defined openings and controlled pouring. Furthermore, detailed and fine tamper evidence features can be achieved with the containers of the present invention.

### Brief Description of the Drawings

- Figure 1: is a schematic drawing showing a container for irrigation application according to the present invention.
- Figure 2: is a schematic drawing showing a break-off cap of the container according to the present invention.
- Figure 3: shows a schematic view of a cross-section of the break-off cap of the container according to the present invention.
- Figure 4: shows a schematic view of a cross-section of the break-off cap of the container according to the present invention.

### Reference signs

The following reference signs are used throughout the description and the figures.
- **1**: container for irrigation application
- **2**: base container
- **3**: at least one wall
- **4**: opening
- **5**: break-off cap
- **6**: seal
- **7**: flange of the opening
- **8**: flange of the break-off cap
- **9**: predetermined breaking area
- **10**: top of the cap
- **11**: irrigation jet of the cap
- **12**: opening lever of the cap
- **13**: bar of the opening lever
- **14**: tamper evident means of the cap
- **15**: gripping means of the bar

### Definitions

The term *"irrigation solution"* as used herein denotes a liquid used in irrigation application. Irrigation solutions have to be sterile. An irrigating solution is suitable for killing microorganisms, disrupting the biofilm on surgery objects, inactivating virulence factors such as endotoxin, dissolving pulp-tissue remnants, removing hard-tissue debris and the smear layer created during instrumentation or prevent their formation, providing lubrication for instruments, and being biocompatible. Usually, an irrigation solution is based on water. Optionally, it can be mixed with an adjuvant. Adjuvants may be sodium chloride, sodium hypochlorite, potassium chloride, calcium chloride, chlorhexidine, ethylenediamine tetraacetic acid, citric acid, etidronic acid, maleic acid, sodium lactate, or mixtures thereof. Most preferably, irrigation solutions are selected from the list consisting of 0.9% aqueous sodium chloride solution, Ringer's lactate solution, and distilled water.

The term *"surgery object"* as used herein denotes an object, preferably body part, which can be treated by surgery, wherein the body part is preferably selected from a group consisting of body cavities, tissues, wounds, bones, and burns.

The term *"irrigation"* or *"irrigation application"* as used herein denotes the process of mechanical cleansing of surgery objects during topical, intra- or postoperative surgical interventions using an irrigation solution. This is typically achieved by pouring the irrigation solution on the surgery object and rinsing it, which is also known as *"direct irrigation".* Alternatively, such a process is achieved by pouring out liquid in an intermediate recipient (like a kidney bowl), from which the solution is then taken out with a syringe to be spread onto the object, which is also known as *"indirect irrigation".* The goal of the irrigation is to clear a surgical object from, e.g., blood and tissue to keep the surgery object sterile, prevent infections, moisten the surgery object, wound tamponades, cloths, bandages, and dressings, cleansing of operating instruments and accessories, and/or make the object visible for the surgeon. It is understood that prior to and/or during the process of irrigation, the container comprising the irrigation solution has to be put aside, at least one time.

The term *"upright position"* as used herein denotes an orientation of the bag of the present invention in that each distance of each point in the base part of the bag to the center of the gravitational force is smaller than in any point of the upper part and optionally the top part of the bag.

The term *"used in an upright position"* as used herein in particular in connection with the irrigation application denotes an irrigation process, in which the container is used such that the container is in upright position when being put aside.

The term *"sterile"* as used denotes the status of an object having a significantly reduced number of bacteria and/or viruses on its surface to reduce the risk of an infection. In particular, the term *"sterile"* denotes an object or substance, which has a bioburden load of lower than 10⁻⁶. The bioburden load can be measured i.e., according to ISO 11737-1:2018.

The term *"sterilization"* as used herein denotes a method to destroy all forms of living microorganisms from a substance. As there is always a certain probability of at least one microorganism to survive such procedure, the aim of sterilization is the reduction of initially present microorganisms or other potential pathogens. Generally, sterilization is accepted to be achieved if the bioburden load of the substance of object to be sterilized is lower than 10⁻⁶. The bioburden load can be measured i.e., according to ISO 11737-1:2018. Sterilization can be achieved using several methods. In one sterilization process the object is heated up to at least 105 °C to achieve a sterile object. Thereby, the object should not be deformed by the elevated temperature. Preferably, the heating step is performed in an autoclave. In another sterilization process, the object is brought into contact with toxic gases such as a mixture of ethylene oxide and carbon dioxide. Filtration methods are also used to sterilize liquids, i.e., by using membrane filters, Seitz filters, and/or candle filters. Finally, sterilization can be achieved by indirect energy import into or onto the object, e.g., by ultrasonic waves, ultraviolet light, as well as by high energy particles (such as electrons, gamma- or X-rays).

The term "*liquid-tight*" as used herein denotes a quality of an object to function as a barrier for a liquid, preferably for a water-based liquid.

The term *"oval shape"* as used herein denotes a plane curve, which is simple, i.e., not self-intersecting, convex, and closed. Preferably, an oval shape has at least one symmetry axis, more preferably two symmetry axes, and most preferably two symmetry axes, wherein the angle between these two symmetry axes is a rectangular angle. Specific oval shapes are circles, ellipsoids, rectangles with quadrant corners, and stadiums.

The term *"rectangular-oval shape"* as used herein denotes a rectangle with quadrant corners or a stadium shape, preferably a rectangle with quadrant corners.

The term *"cylindrical shape"* or *"cylinder shape"* as used herein denotes a shape of all points on all lines which are parallel to a given main axis and which pass through a fixed plane curve in a plane not parallel, preferably perpendicular, to the main axis, wherein the main axis preferably passes through the centroid of the fixed plane curve. The lines have a defined length, which defines the length of the cylindrical shape. Hence, in a cylindrical shape, the fixed plane curve is identical at both ends. Most preferably, the main axis of the fixed plane curve is the circular symmetry axis of the fixed plane curve.

The term *"break-off cap"* denotes a cap, which can be opened by breaking of at least one part thereof. Preferably, the break-off cap comprises a tube, which is closed at the end extending from the container. This tube preferably has a recess, preferably a circular recess having the same symmetry axis as the tube. In this recess, the material thickness is lower than the material thickness of the wall of the tube, preferably lower than 0.1 times the thickness of the wall of the tube. Preferably, the term break-off cap as used herein denotes caps, which are broke off by twisting the at least one part thereof, thereby opening the cap.

### Detailed Description of the Invention

In the following, the present invention will be described in detail. Thus, the process for producing the container of the present invention, the container of the present invention, the use of the container of the present invention, and the method for irrigation a surgery object using a container according to the present invention are described in the following.

### Process

As outlined above, the present invention is directed towards a process for producing a container for irrigation application (**1**), comprising the steps of
- providing a base container (**2**) comprising at least one wall (**3**), wherein the at least one wall (**3**) comprises an opening (**4**);
- providing a break-off cap (**5**);
- fluidly connecting in a connecting step the break-off cap (**5**) with the opening (**4**) of said base container (**2**) forming a connection of the opening (**4**) with the break-off cap (**5**);
- sealing in a sealing step the connection of the opening (**4**) with the break-off cap (**5**).

Generally, the base container (**2**) can be formed by any process step known in the prior art, wherein the process step provides a base container (**2**) according to the present invention, i.e., a base container (**2**) comprising, preferably consisting of, a polymer material, preferably a polymer material selected from the list consisting of polyethylene, polypropylene, polyethylene terephthalate, polyamide, ethylene vinyl alcohol, or mixtures thereof, most preferably polyethylene terephthalate. Reason is that a polymer material can provide the necessary physical properties in view of transparency, impact properties, and flexibility. Furthermore, such materials are appropriate for being sealed. Hence, preferably, the step of providing the base container (**2**) comprises the step of molding a base container (**2**), preferably blow-molding a base container (**2**) of the present invention. Most preferably, the base container (**2**) is blow-molded from a tube comprising, preferably consisting of a polymer material, preferably a polymer material selected from the list consisting of polyethylene, polypropylene, polyethylene terephthalate, polyamide, ethylene vinyl alcohol, or mixtures thereof, most preferably polyethylene terephthalate.

Generally, the break-off cap (**5**) can be formed by any process step known in the prior art, wherein the process step provides a break-off cap (**5**) according to the present invention, i.e., a break-off cap (**5**) comprising, preferably consisting of, a polymer material, preferably a polymer material selected from the list consisting of polyethylene, polypropylene, polyethylene terephthalate, polyamide, ethylene vinyl alcohol, or mixtures thereof, most preferably polyethylene terephthalate. Reason is that a polymer material can provide the necessary physical properties in view of transparency, impact properties, and flexibility. Furthermore, such materials are appropriate for being sealed. Hence, preferably, the step of providing the break-off cap (**5**) comprises the step of molding a break-off cap (**5**), preferably injection-molding a break-off cap (**5**) of the present invention. Most preferably, the break-off cap (**5**) is blow-molded from a parison comprising, preferably consisting of a polymer material, preferably a polymer material selected from the list consisting of polyethylene, polypropylene, polyethylene terephthalate, polyamide, ethylene vinyl alcohol, or mixtures thereof, most preferably polyethylene terephthalate.

More preferably, the polymer material of the base container (**2**) has a haze value measured according to ASTM D1003, Procedure B, of less than 30%, preferably less than 20%, more preferably less than 10%, and most preferably less than 5%. This helps that the person using the container can see from the outside how much solution is left in the container.

By sealing the break-off cap (**5**) with the opening (**4**), the container (**1**) is liquid-tightly closed. Hence, in case a filled container is produced, the process preferably comprises a filling step of filling the base container (**2**) with an irrigation solution prior to the connecting step and subsequent to the step of providing the base container (**2**). Preferably, the filling step is carried out by holding the base container in a holding means and filling the irrigation solution by a filling jet. As the filled container is usually sterilized after sealing, this step does not necessarily need to be a sterile step. Hence, preferably, the process of the present invention comprises a sterilization step subsequent to the sealing step.

Preferably, the step of sealing the connection comprises the step of sealing the connection using a seal (**6**), preferably an overmold ring (**6**). A seal has the advantage that materials can be sealed, which are not compatible in terms of direct sealing, i.e., melting the materials together. The overmold ring has the advantage that not only the gap between the break-off cap (**5**) and the opening (**4**) is sealed, but the overmold ring extends therefrom on the area close to the gap. This ensures better connection and seal strength.

In a preferred embodiment of the present invention, the opening (**4**) is formed by a cylindrical tube in and extending from the at least one wall (**3**) and the break-off cap (**5**) comprises a cylindrical tube, wherein the inner diameter of the cylindrical tube of the break-off cap (**5**) is larger than the outer diameter of the cylindrical tube of the opening (**4**), and wherein the connecting step comprises putting the cylindrical tube of the break-off cap (**5**) over the cylindrical tube of the opening (**4**). This has the advantage of a facilitated process step, as the break-off cap (**5**) can simply be into the opening (**4**), whereby the break-off cap (**5**) and the opening (**4**) are assembled, thereby forming the fluid connection. In an even more preferred embodiment of the process of the present invention, the inner surface of the cylindrical tube of the break-off cap (**5**) comprises a thread and the outer surface of the cylindrical tube of the opening (**4**) comprises a counter thread to said thread, and wherein the connecting step comprises the step of assembling the cylindrical tube of the break-off cap (**5**) on the cylindrical tube of the opening (**4**). These embodiments allow for quick, facile, and tight connection of the break-off cap (**5**) and the opening (**4**).

The cylindrical tube of the break-off cap (**5**) preferably comprises a flange (**7**), more preferably at the edge of the cylindrical tube, and the cylindrical tube of the opening (**4**) comprises a flange (**8**) at the outer surface, and wherein the sealing step comprises the step of sealing the connection by sealing the flange (**7**) of the cylindrical tube of the opening (**4**) and the flange (**8**) of the cylindrical tube of the break-off cap (**5**), preferably by using an overmold ring (**6**). In such an embodiment, the area of the connection between the break-off cap (**5**) and the opening (**4**) is enlarged and thus the sealing strength is improved. Furthermore, the seal, preferably the overmold ring (**6**), can be attached in a more facile way, as the area for overlapping is enlarged by the flanges (**7**) and (**8**).

### Container

As also outlined above, the present invention is concerned with a container for irrigation application (**1**), the container comprising
- a base container (**2**) comprising at least one wall (**3**), wherein the at least one wall (**3**) comprises an opening (**4**),
- a break-off cap (**5**) fluidly connected to said opening (**4**),
- a seal (**6**) between the break-off cap (**5**) and the base container (**2**).

The container according to claim 8, wherein the seal (**6**) is an overmold ring (**6**).

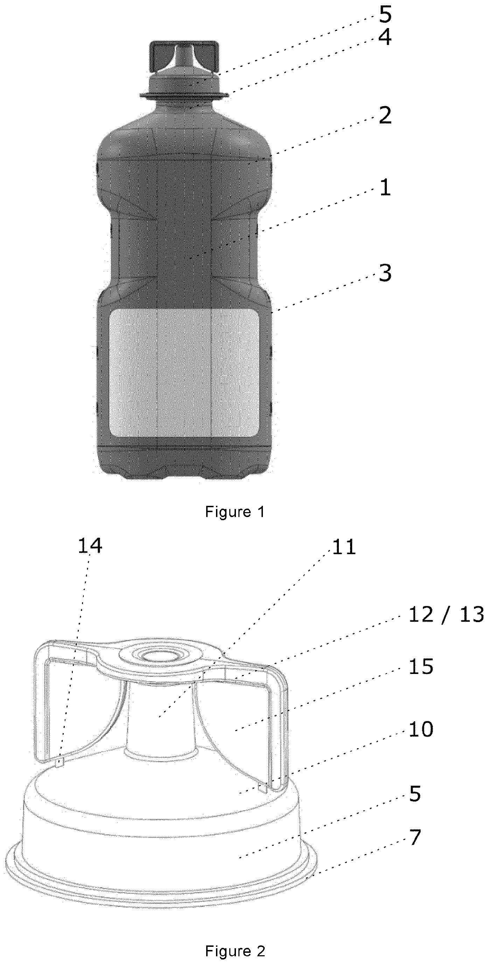

A container according to the present invention is shown in Figure 1.

### a) Opening

In the container according to the present invention, the opening (**4**) is preferably formed by a cylindrical tube in the at least one wall (**3**), which extends from the at least one wall (**3**), and the break-off cap (**5**) comprises a cylindrical tube, wherein the inner diameter of the cylindrical tube of the break-off cap (**5**) is larger than the outer diameter of the cylindrical tube of the opening (**4**), and wherein the cylindrical tube of the break-off cap (**5**) is put over the cylindrical tube of the opening (**4**).

Preferably, the inner surface of the cylindrical tube of the break-off cap (**5**) comprises a thread and the outer surface of the cylindrical tube of the opening (**4**) comprises a counter thread to said thread, and wherein the cylindrical tube of the break-off cap (**5**) is screwed on the cylindrical tube of the opening (**4**).

Also preferably, the cylindrical tube of the break-off cap (**5**) comprises a flange (**7**), preferably at the edge of the cylindrical tube, and the cylindrical tube of the opening (**4**) comprises a flange (**8**) at the outer surface. In parallel to the description as provided for the process of the present invention, these embodiments allow for quick, facile, and tight connection of the break-off cap (**5**) and the opening (**4**). Furthermore, the seal, preferably the overmold ring (**6**), can be attached in a more facile way, as the area for overlapping is enlarged by the flanges (**7**) and (**8**).

### b) Seal

Hence, as already disclosed for the process of the present invention, the container according to claim 12, wherein the flange (**7**) of the cylindrical tube of the opening (**4**) and the flange (**8**) of the cylindrical tube of the break-off cap (**5**) are sealed by the overmold ring (**6**). The seal, preferably the overmold ring (**6**), comprises, preferably consists of, a polymer material, which either shrinks at elevated temperature, preferably temperatures higher than 22 °C, more preferably higher than 200 °C; and usually at temperatures lower than 270 °C, or which melts at temperatures higher than 200 °C; and usually at temperatures lower than 270°C. Most preferably, the material of the seal, preferably overmold ring (**6**), comprises, preferably consists of, a polymer material, more preferably a polyolefin, most preferably a polyethylene or polypropylene.

### c) Break-off cap

The break-off cap (**5**) preferably comprises a predetermined breaking area (**9**), wherein the predetermined breaking area (**9**) is preferably an area, in which the thickness of a wall of the break-off cap (**5**) reaches a minimum. Usually, the thickness of the walls depends on the material used for the walls of the break-off cap. Preferably, the break-off cap (**5**) comprises, preferably consists of, a polymer material, preferably a polymer material selected from the list consisting of polyethylene, polypropylene, polyethylene terephthalate, polyamide, ethylene vinyl alcohol, or mixtures thereof, most preferably polyethylene terephthalate. For polyolefins such as low density polyethylene (**LDPE**), high density polyethylene (**HDPE**), and/or polypropylene (**PP**), the thickness of the wall usually is in the range of from 0.1 mm to 0.5 mm. Hence, preferably, the predetermined breaking area (**9**) has a thickness in the range of from 0.01 m to 0.05 mm.

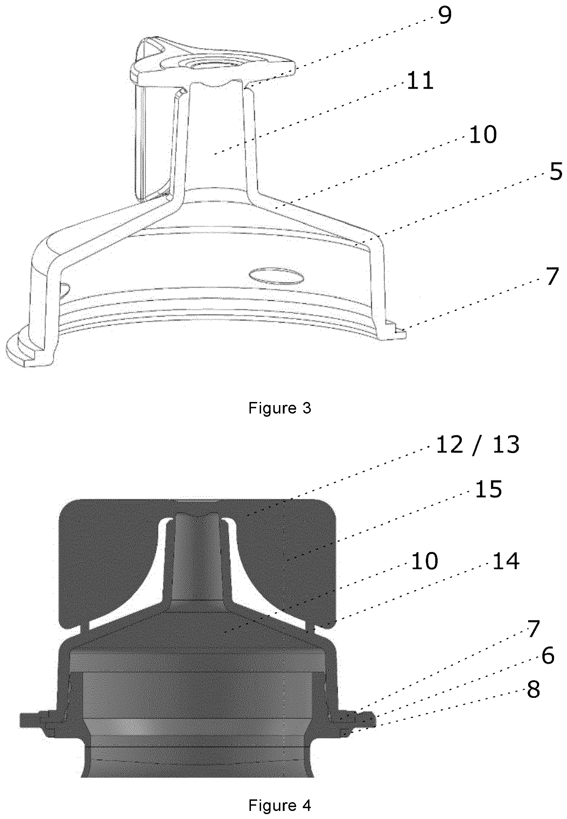

A preferred embodiment of the break-off cap according to the present invention is depicted in Figures 2 and 4. The cylindrical tube of the break-off cap (**5**) preferably comprises a top (**10**), wherein the top (**10**) comprises an irrigation jet (**11**), wherein the irrigation jet (**11**) has a cone shape, and an opening lever (**12**), wherein the opening lever (**12**) is positioned at the top (**10**) of the cone-shaped irrigation jet (**11**), thereby closing the opening (**4**) of the cone-shaped irrigation jet (**11**), cf. Figures 2 and 3. This embodiment has the advantage that the break-off cap can be conveniently opened by twisting the opening lever (**12**). Preferably, the top (**10**) of the cylindrical tube of the break-off cap (**5**) has a dome shape (**10**) and the irrigation jet (**11**) is positioned at the maximum of the dome shape (**10**). This enhances the pouring handling of the container (**1**) and further facilitates processing due to the symmetry of the break-off cap (**5**).

More preferably, the predetermined breaking area (**9**) is positioned between the opening (**4**) of the cone-shaped irrigation jet (**11**) and the opening lever (**12**) as has been shown in Figure 3. This further helps to twist-break the break-off cap (**5**) leaving a well-defined circular opening for precise irrigation.

As can be seen in Figure 4, the opening lever (**12**) preferably comprises a bar (**13**), wherein each end of the bar (**13**) extends in a 90° angle relatively to the axis of the cone-shaped irrigation jet (**11**). More preferably, the distances from each end of the bar (**13**) to the axis of the cone-shaped irrigation jet (**11**) are identical, preferably not larger than the radius of the cylindrical tube of the break-off cap (**5**).

Furthermore, preferably, the opening lever (**12**) comprises gripping means, wherein the gripping means (**15**) extend from the bar (**13**) towards the distances from each end of the bar (**13**) parallel to the axis of the cone-shaped irrigation jet (**11**) towards the cylindrical tube of the top (**10**) of the cylindrical tube of the break-off cap (**5**). Even more preferably, the gripping means (**15**) are divided by a gap from the surface of the irrigation jet (**11**) and preferably from at least a part of the surface of the top (**10**) of the cylindrical tube of the break-off cap (**5**). This allows a maximum area of the gripping means (**15**) for applying pressure by the fingers without hindering the breaking mechanism of the predetermined breaking area (**9**).

In another preferred embodiment of the present invention, the break-off cap (**5**) comprises a tamper evident closure (**14**). Preferably, the tamper evident closure (**14**) is a bridge connecting the gripping means (**15**) connected with a part of the surface of the top (**10**) of the cylindrical tube of the break-off cap (**5**), wherein the bridge has a width smaller than the average width of the wall of the break-off cap (**5**), preferably the width of the predetermined breaking area (**9**). Such a small tamper evident closure is very sensitive to any movement of the opening lever. Hence, even very small movements, which would possibly not completely break, but only crack the predetermined breaking area (**9**) leaving non-visible cracks therein could be determined by such tamper evident closures (**14**). It should be noted that such small features need to be produced by a process able to achieve precise molding. Hence, for example, forces applied to the lever during transport can be detected.

In another preferred embodiment, at least a part of the bar (**13**) of the opening lever (**12**) has a width larger than the diameter of the opening (**4**) of the cone-shaped irrigation jet (**11**), preferably centered around the axis of the cone-shaped irrigation jet (**11**). An embodiment thereof is shown in Figure 3. Such an embodiment has the advantage that the opening, which is created after breaking the predetermined breaking area (**9**), is protected against contamination, for example by the hand of the person, which opens the container.

Preferably, the shape of the base container (**2**) is a cylinder, whereas the base area of the cylinder has an oval shape. Preferably, the oval shape is a shape selected from the list consisting of an ellipsoid, a rectangle with quadrant corners, and a stadium shape, more preferably is a stadium.

In a preferred embodiment of the present invention, the base container is a filled base container. Preferably, the base container is filled with an irrigation solution. More preferably, the filled container has been sterilized.

### Use

Furthermore, the present invention is directed towards the use of the container according to the present invention in an irrigation application.

### Method for irrating

Finally, the present invention is concerned with a method for irrigating an object during surgery with a container according to the present invention.

## Claims

1. A process for producing a container for irrigation application (**1**), comprising the steps of
- providing a base container (**2**) comprising at least one wall (**3**), wherein the at least one wall (**3**) comprises an opening (**4**);
- providing a break-off cap (**5**);
- fluidly connecting in a connecting step the break-off cap (**5**) with the opening (**4**) of said base container (**2**) forming a connection of the opening (**4**) with the break-off cap (**5**);
- sealing in a sealing step the connection of the opening (**4**) with the break-off cap (**5**).

2. The process according to claim 1, wherein the step of sealing the connection comprises the step of sealing the connection using a seal (**6**), preferably an overmold ring (**6**).

3. The process according to claims 1 or 2, wherein the opening (**4**) is formed by a cylindrical tube in and extending from the at least one wall (**3**) and the break-off cap (**5**) comprises a cylindrical tube, wherein the inner diameter of the cylindrical tube of the break-off cap (**5**) is larger than the outer diameter of the cylindrical tube of the opening (**4**), and wherein the connecting step comprises putting the cylindrical tube of the break-off cap (**5**) over the cylindrical tube of the opening (**4**).

4. The process according to any of claims 3, wherein the cylindrical tube of the break-off cap (**5**) comprises a flange (**7**), preferably at the edge of the cylindrical tube, and the cylindrical tube of the opening (**4**) comprises a flange (**8**) at the outer surface, and wherein the sealing step comprises the step of sealing the connection by sealing the flange (**7**) of the cylindrical tube of the opening (**4**) and the flange (**8**) of the cylindrical tube of the break-off cap (**5**), preferably by using an overmold ring (**6**).

5. The process according to any of the preceding claims, wherein the base container (**2**) is filled with an irrigation liquid in a filling step prior to the connecting step.

6. A container for irrigation application (**1**), the container comprising
- a base container (**2**) comprising at least one wall (**3**), wherein the at least one wall (**3**) comprises an opening (**4**),
- a break-off cap (**5**) fluidly connected to said opening (**4**),
- a seal (**6**) between the break-off cap (**5**) and the base container (**2**).

7. The container according to claim 6, wherein the seal (**6**) is an overmold ring (**6**).

8. The container according to any of claims 6 or 7, wherein the opening (**4**) is formed by a cylindrical tube in the at least one wall (**3**), which extends from the at least one wall (**3**), and the break-off cap (**5**) comprises a cylindrical tube, wherein the inner diameter of the cylindrical tube of the break-off cap (**5**) is larger than the outer diameter of the cylindrical tube of the opening (**4**), and wherein the cylindrical tube of the break-off cap (**5**) is put over the cylindrical tube of the opening (**4**).

9. The container according to claim 8, wherein the inner surface of the cylindrical tube of the break-off cap (**5**) comprises a thread and the outer surface of the cylindrical tube of the opening (**4**) comprises a counter thread to said thread, and wherein the cylindrical tube of the break-off cap (**5**) is screwed on the cylindrical tube of the opening (**4**).

10. The container according any of claims 8 or 9, wherein the cylindrical tube of the break-off cap (**5**) comprises a flange (**7**), preferably at the edge of the cylindrical tube, and the cylindrical tube of the opening (**4**) comprises a flange (**8**) at the outer surface.

11. The container according to claim 10, wherein the flange (**7**) of the cylindrical tube of the opening (**4**) and the flange (**8**) of the cylindrical tube of the break-off cap (**5**) are sealed by the overmold ring (**6**).

12. The process according to any of the preceding claims 1 to 5 or the container according to any of the preceding claims 6 to 11, wherein the break-off cap (**5**) comprises a predetermined breaking area (**9**), wherein the predetermined breaking area (**9**) is preferably an area, in which the thickness of a wall of the break-off cap (**5**) reaches a minimum.

13. The process according to any of the preceding claims 1 to 5 and 12 or the container according to any of the preceding claims 6 to 12, wherein the break-off cap (**5**) comprises a tamper evident closure (**14**).

14. The use of the container according to any of the preceding claims 6 to 12 in an irrigation application.

15. A method for irrigating an object during surgery with a container according to any of the preceding claims 6 to 12.
